# EUROPÄISCHE PATENTSCHRIFT

(11) **EP 3 429 484 B1**
(45) Veröffentlichungstag und Bekanntmachung des Hinweises auf die Patenterteilung: **12.05.2021**
(21) Anmeldenummer: 17709692.2
(22) Anmeldetag: 09.03.2017
(51) Int. Cl.: A61B 17/15

(54) **OPERATIONSVORRICHTUNG FÜR EINE OPERATION DES MENSCHLICHEN KNIES**
SURGICAL DEVICE FOR SURGERY OF THE HUMAN KNEE
DISPOSITIF D'INTERVENTION POUR UNE OPÉRATION DU GENOU HUMAIN

(30) Priorität: 16.03.2016 DE 102016204307
(43) Veröffentlichungstag der Anmeldung: 23.01.2019
(73) Patentinhaber: Boos, Carsten, 9403 Goldach (CH)
(72) Erfinder: Boos, Carsten, 9403 Goldach (CH)
(74) Vertreter: Eisenführ Speiser
(86) Internationale Anmeldenummer: PCT/EP2017/055598
(87) Internationale Veröffentlichungsnummer: WO 2017/157767

(56) Entgegenhaltungen:
- EP-A2- 0 809 969
- EP-A2- 1 348 382
- WO-A1-2011/135372
- WO-A2-2006/042743
- GB-A- 2 526 724
- US-A- 5 662 656
- US-A1- 2005 256 527
- US-A1- 2006 189 998
- US-A1- 2006 241 634
- US-A1- 2012 259 342
- US-A1- 2015 209 158

## Beschreibung

Die vorliegende Erfindung betrifft eine Operationsvorrichtung. Insbesondere betrifft die vorliegende Erfindung eine Operationsvorrichtung, die bei der Durchführung einer Operation eines menschlichen Knies, insbesondere bei einer Implantation einer Knie-Endoprothese, zum Einsatz kommt.

Das Kniegelenk eines Menschen ermöglicht die Bewegung zwischen Oberschenkel (Femur) und Unterschenkel, nämlich Wadenbein (Fibula) und Schienbein (Tibia). Auf der vorderen Seite des Kniegelenks bildet die Kniescheibe (Patella) einen weiteren Gelenkpartner. Die Ungleichheit der Gelenkflächen zwischen Femur und Tibia wird durch Knorpelscheiben, genannt Menisken, ausgeglichen. Bei schwerem Verschleiß des Knies, der sogenannten Kniegelenksarthrose, beziehungsweise nach Verletzungen des Knies kann eine vollständige Prothese des Kniegelenks notwendig werden.

Das Kniegelenk wird durch seine zwei Seitenbänder, nämlich ein inneres und ein äußeres, genannt Ligamente, in seitlicher Richtung stabilisiert, damit ein Wegknicken in eine O-Beinstellung oder eine X-Beinstellung verhindert wird. Das vordere und hintere Kreuzband in der Mitte des Kniegelenkes begrenzen die Verschiebung des Femurs und der Tibia in der Saggitalebene.

Eine Knie-Endoprothese besteht im Allgemeinen aus zwei Metallkomponenten, die jeweils in den Femur und die Tibia zementiert oder zementfrei verankert werden sowie einer dazwischenliegenden Gleitkomponente, insbesondere aus Polyethylen. Für den Dreh-Gleit-Mechanismus sind mehrere Verfahren bekannt.

Für die Implantation einer Knieendoprothese sind verschiedenste Operationstechniken beschrieben. In der Regel werden zunächst die Stirnfläche von Femur und Tibia abgesägt und geglättet. Dabei sollte eine vorbestehende Achsfehlstellung (O- oder X-Bein) unter Berücksichtigung der korrekten Bandspannung korrigiert werden. Sind die Stirnflächen vorbereitet, werden anschließend die definitive Femurrotation entweder anatomisch orientiert, ligamentbalanciert oder kombiniert präpariert. Bei den ligamentbalancierten Verfahren wird mithilfe unterschiedlicher Spannvorrichtungen im gebeugten (und zum Teil auch gestrecktem) Zustand des Knies die Ligamente gleichmäßig gespannt und mittels Bohrschablonen, die in der Spannvorrichtung enthalten sind, die Position der danach einzusetzenden Vorrichtung festgelegt. Durch diesen Operationsschritt ist zwar eine korrekte Rotation der Femurprothese erreicht, aber für die korrekte Größenbestimmung der Femurkomponente in der Saggital- und Frontalebene (parallel hierzu muss auch die Tibiakomponente größenadaptiert werden) muss in einem weiteren Schritt nach Abnahme der Spannvorrichtung ein Lehrenblock, welcher ein Ablehren der Schneidebenen an der Ober-/Unterseite des Femur ermöglicht, an die Stirnseite des Femur mittels der vorher vorbereiteten Bohrungen befestigt werden. In einem Winkel zu der Femurstirnfläche werden dann die Ober- und Unterseite des Femur abgelehrt und bei Bedarf vermessen.

Mit dem Begriff Lehren bzw. Ablehren wird eine visuelle Prüfung und ein Ausmessen der Ober- und Vorderseite des Femur bezeichnet, was gegebenenfalls ein Vermessen mit einer Fühlerlehre, beispielsweise einer blechartigen Fühlerlehre, umfasst.

Im Anschluss daran wird der Lehrenblock entfernt und je nach Größe der Prothese unterschiedliche Prothesen-Bohrschablonen auf einen Schnittblock separat aufgesetzt. Die Bohrungen werden ausgeführt und die Vorrichtung(en) entfernt. In die ausgeführten Bohrungen wird ein nicht dargestellter Schnittblock separat aufgesetzt. Mithilfe weiterer Prothesen-Bohrschablonen werden dann die zwei endgültigen Bohrungen für die Femurprothese gesetzt.

Das Einspannen der Spannvorrichtung mitsamt Vorsehen der Hilfsbohrungen, daran anschließend das Befestigen des Lehrenblockes mit Ablehren der Schneidebenen und daran anschließendes Anbringen der Prothesen-Bohrschablonen erfordert drei Vorrichtungen, die jeweils aufwendig und fehleranfällig zu montieren und zu demontieren sind. Für die korrekte Bedienung der Vorrichtungen ist ein hohes Maß an Erfahrung nötig, was insbesondere jungen, unerfahrenen Operateuren Probleme bereiten kann. Ferner ist die Arbeit mit mehreren Vorrichtungen zeitintensiv, was die Operation allgemein teurer macht, und das Operations- und Infektionsrisiko erhöht.

Aus dem Stand der Technik ist beispielsweise die Knochenschnittführungseinheit der EP 0 809 969 B1 bekannt. Die Spannvorrichtung wird in einem gestreckten Zustand des Knies, wie in Fig. 6 gezeigt, eingesetzt und Hilfsbohrungen 54 in dem Femur, nicht aber auf der Femurstirnfläche, vorgesehen. In mehreren Schritten wird dann, nach Entfernen der Vorrichtung, wie beispielsweise in Figuren 7 und 9 gezeigt, zunächst eine Größe der Prothese bestimmt. Das Bohren der Befestigungslöcher ist dann, wie in Fig. 16 gezeigt, nach einem erneuten Einsetzen der Vorrichtung möglich. Durch die vielen bei dem Operationsprozess notwendigen Schritte und die komplizierte Durchführung der Operation kommt es zu einer zeitintensiven, teureren und risikoreichen Operation. Des weiteren kommt es durch die Zwangslage, welche durch die Gewebeeingriffsoberflächen 3 und 6 erzwungen wird, zu einer gegenüber einer momentenbalancierten Ausrichtung des Femurs zu einer ein größeres Fehlerpotential aufweisenden Spannung der Ligamente.

Aus EP 1 348 382 B1 ist eine Vorrichtung zum Einstellen des Beugeabstandes einer Knieprothese bekannt. Das Instrument weist ein Basisteil, das für die Anordnung relativ zu dem proximalen Ende des Schienbeins ausgestaltet ist, eine Platte, die von dem Basisteil hervorsteht und so ausgestaltet ist, dass sie relativ zu dem distalen Ende des Oberschenkels an diesem liegend ausgestaltet ist, wenn das Basisteil relativ zu dem Schienbein angeordnet ist, und Markierungen auf der Platte auf, welche eine Spaltabmessung definieren. Die Platte ist ein Teil eines aufragenden Bereiches eines I-Trägers, der von dem Basisteil hervorsteht. Dieses Instrument erfordert die Auswahl eines passenden Schneidblockes durch Probieren, bevor die Bohrungen für den Schnittblock gesetzt werden können, was die Operation ebenfalls zeitintensiv und fehleranfällig gestaltet.

Es ist somit eine Aufgabe der vorliegenden Erfindung, eine Vorrichtung zur Operation von Knie-Endoprothesen bereitzustellen, welche die Sicherheit des Durchführens der Operation verbessert.

Die Aufgabe wird erfindungsgemäß durch eine Operationsvorrichtung für eine Operation eines menschlichen Knies gelöst, wobei die Operationsvorrichtung eine Führungskomponente, eine Spannkomponente und eine Ablehr- und Bohrkomponente aufweist. Die Führungskomponente weist einen Grundkörper mit einer Grundfläche zur Anordnung auf einer Tibiastirnfläche und sich von der Grundfläche erstreckende Führungselemente auf. Die Spannkomponente ist dazu eingerichtet, die Führungskomponente derart zu spannen, dass die Ligamente des Knies in einem gebeugten Zustand des Knies gleichmäßig gespannt sind. Eine Ablehr- und Bohrkomponente zum Ablehren und Bohren der Femurstirnfläche ist auf die Führungselemente aufschiebbar. Die Ablehr- und Bohrkomponente ist in verschiedenen Positionen bezüglich der Grundfläche fixierbar. Die Ablehr- und Bohrkomponente weist einen Lehrenaufsatz auf, wobei der Lehrenaufsatz wenigstens eine Nut für einen oberen Femurschnitt aufweist. Der Lehrenaufsatz weist mehrere Durchgangsöffnungen auf und das Führungselement weist mehrere jeweils zugehörige Aussparungen auf, wobei einander zugehörige Durchgangsöffnungen und Aussparungen die gleiche Orientierung aufweisen und wobei voneinander verschiedene Durchgangsöffnungen voneinander verschiedene Orientierungen aufweisen.

Die erfindungsgemäße Operationsvorrichtung kommt dann während der Operation zum Einsatz, wenn die Femurstirnfläche und die Tibiastirnfläche vorbereitet sind. Die Operationsvorrichtung wird in das gebeugte Knie eingeführt, wobei die Grundfläche des Grundkörpers auf der Tibiastirnfläche aufliegt. Die sich von der Grundfläche erstreckenden Führungselemente verlaufen dann parallel zu der Femurstirnfläche und sind eingerichtet, mit dieser ausgerichtet zu sein. Dadurch, dass eine Ablehr- und Bohrkomponente auf die Führungselemente aufschiebbar ist, ist eine fehlerhafte Positionierung des bisher verwendeten Lehrenblockes nicht möglich. Darüber hinaus entfällt der Zwischenschritt des Entspannens der Spannvorrichtung sowie des Entfernens der Spannvorrichtung, wodurch auch der Operationsaufwand verringert wird. Damit können, durch eine verkürzte Operationszeit, Komplikationen für den Patienten minimiert werden.

Die erfindungsgemäße Operationsvorrichtung ist demnach dazu eingerichtet, dass ohne die Spannvorrichtung zu lösen, ein Ablehren und Bohren des Femur möglich ist. Somit wird das Operationsverfahren vereinfacht, da weniger Vorrichtungen einzusetzen sind.

Indem die Ablehr- und Bohrkomponente auf die Führungselemente aufschiebbar ist, kann die Femurstirnfläche abgelehrt werden, ohne dass die Spannkomponente und die Führungskomponente entspannt beziehungsweise von dem Femur entfernt werden müssen.

Somit kann das Ablehren und Bohren mit geringem Aufwand und verringertem Fehlerrisiko durchgeführt werden.

Indem die Ablehr- und Bohrkomponente in verschiedenen Positionen bezüglich der Grundfläche fixierbar ist, kann die Ablehr- und Bohrkomponente nahezu unabhängig von der Größe des Knies universell eingesetzt werden.

Die Stirnfläche des Femur beziehungsweise der Tibia sind die Flächen, die sich im Kniegelenk gegenüberstehen. Als Vorder- beziehungsweise Oberseiten der jeweiligen Knochen werden diejenigen Seiten bezeichnet, die auf Seite der Patella im Kniegelenk sind. Mit anderen Worten, die Seite, die üblicherweise bei einem Menschen als vorne bezeichnet wird. Entsprechend sind Hinterseiten des Femur beziehungsweise der Tibia diejenigen Seiten, die an die Kniekehle angrenzen.

Hierdurch wird das Problem gelöst, dass ein Operateur die Größe der Prothese und die Lage der weiteren Sägeschnitte zunächst bestimmen musste, bevor die Prothesenbohrungen in den Femur gebohrt werden. Erfindungsgemäß kann durch die Ablehr- und Bohrkomponente das Ablehren und Bohren mit einem einzigen auf den Führungselementen aufgesetzten und festgestellten Ablehraufsatz erreicht werden.

In einer Ausführungsform umfasst die Spannkomponente einen Spannzylinder und einen Spannhebel. Der Spannzylinder ist von den Führungselementen entlang einer Längsachse des Spannzylinders geführt und der Spannhebel ist dazu eingerichtet, den Spannzylinder bezüglich des Grundkörpers zu spannen.

Vorzugsweise ermöglicht die Spannkomponente somit, dass die Operationsvorrichtung auf einen definierten Abstand zwischen Spannzylinder und Führungskomponente beziehungsweise Grundkörper gespannt werden kann. Beispielsweise kann der Spannzylinder bezüglich des Femur fixiert sein und der Grundkörper in Richtung der Tibiastirnfläche bewegt werden, bis die gewünschte Spannung erreicht ist. Insbesondere kann damit die gleichmäßige Spannung der Ligamente des Knies sichergestellt sein. Der Spannzylinder wird vorzugsweise von den Führungselementen entlang einer Längsachse des Spannzylinders geführt und die Führungselemente gewährleisten die Verschiebbarkeit der Ablehr- und-Bohr-Komponente.

In einer Ausführungsform weist der Spannzylinder auf einer Mantelfläche wenigstens teileweise ein Rastprofil mit mehreren Rastpositionen auf, die dazu eingerichtet sind, dass ein korrespondierendes Rastelement des Spannhebels in mehreren Höhen des Spannzylinders bezüglich des Grundkörpers einrasten kann.

In einer Ausführungsform sind das Rastprofil und das korrespondierende Rastelement derart eingerichtet, dass ein Vergrößern der Entfernung des Spannzylinders von dem Grundkörper ermöglicht und ein Verringern der Entfernung des Spannzylinders von dem Grundkörper blockiert wird.

Vorzugsweise rastet damit der Spannzylinder an mehreren Positionen bezüglich des Spannhebels und des Grundkörpers ein. Indem die Entfernung des Spannzylinders von dem Grundkörper lediglich vergrößert, aber nicht verringert werden kann, wird ein ungewolltes Entspannen der Spannkomponente vermieden.

In einer Ausführungsform weist der Spannzylinder eine Aussparung in Längsrichtung auf. Insbesondere kann die Aussparung in Längsrichtung als eine Durchgangsbohrung in Längsrichtung ausgestaltet sein. Indem der Spannzylinder mit einer Aussparung in Längsrichtung, insbesondere in Form einer irgendwie gearteten Vertiefung oder mit einer Durchgangsbohrung in Längsrichtung, ausgestaltet ist, kann einer Verklemmung aufgrund von Geweben beziehungsweise Flüssigkeiten des Körpers vorgebeugt werden. Damit können Komplikationen bei der Operation weiter vermieden werden.

In einer Ausführungsform weist der Spannzylinder eine oder mehrere Durchgangsbohrungen senkrecht zu der Längsrichtung auf.

Die Durchgangsbohrungen sind vorzugsweise dafür ausgelegt, ein Befestigen und Spannen des Spannzylinders an das Femur mittels eines Halters zu ermöglichen. Der Halterkann beispielsweise in Form eines T-Halters ausgestaltet sein, der ein einfaches Einführen und Lösen des Halters aus dem Femur ermöglicht. Die Mitte des T-Halters ist der mittlere Drehpunkt (Drehlager), der über das Momentengleichgewicht von rechts und links für die gleichmäßige Bandspannung sorgt.

Vorzugsweise weist der Spannzylinder drei Durchgangsbohrungen senkrecht zur Längsrichtung auf, um verschiedenen Größen zu realisieren. Beispielsweise kann die mittlere Durchgangsbohrung für eine durchschnittliche Kniegröße ausgelegt sein, während in Zylinderrichtung näher an dem Grundkörper liegende beziehungsweise weiter davon entfernte Durchgangsbohrungen für größere beziehungsweise kleinere Knie ausgelegt sind. Damit wird eine Einsetzbarkeit der Operationsvorrichtung über einen breiteren Bereich sichergestellt.

In einer Ausführungsform weist der Spannhebel einen ersten Arm und einen zweiten Arm mit einem dazwischenliegenden Drehpunkt auf. Der erste Arm, der zweite Arm, und der dazwischenliegende Drehpunkt sind derart eingerichtet, dass der zweite Arm durch eine Vorspannung des ersten Arms gegen den Spannzylinder gespannt wird.

In einer Ausführungsform weist die Spannkomponente ferner ein Befestigungselement auf. Der Spannhebel ist mittels des Befestigungselementes an seinem Drehpunkt mit dem Grundkörper der Führungskomponente befestigbar. Vorzugsweise ist die Spannkomponente in Form einer Schraube ausgestaltet, wobei in dem Grundkörper ein korrespondierendes Gewinde vorgesehen ist. Der Spannhebel ist damit bezüglich des Grundkörpers lediglich drehbar bewegbar, wobei eine Spannung durch eine Relativbewegung des Spannhebels bzw. des Grundkörpers und des Spannzylinders erfolgt.

In einer Ausführungsform weist die Spannkomponente ferner eine Spannfeder auf, die dazu eingerichtet ist, den ersten Arm bezüglich des Grundkörpers zu spannen. Insbesondere spannt die Spannfeder den ersten Arm bezüglich des Grundkörpers derart, dass der über den Drehpunkt drehbar gelagerte zweite Arm gegen den Spannzylinder gespannt ist.

In einer Ausführungsform weist der Grundkörper auf der Grundfläche eine Aussparung zur Aufnahme des Spannhebels auf. Indem der Spannhebel über eine Aussparung auf der Grundfläche in den Grundkörper eingeführt werden kann, ist es möglich, den Spannhebel nach Benutzung vollständig von dem Grundkörper zu trennen. Damit ist eine gründliche Desinfektion und Wiederverwendbarkeit gewährleistet.

In einer Ausführungsform weist der Grundkörper auf einer Seitenfläche davon eine Öffnung zur Betätigung des Spannhebels auf. Insbesondere kann der Spannhebel gegen die Vorspannung betätigt werden, wodurch der zweite Arm über den Drehpunkt ebenfalls aus der Vorspannung gelöst wird. Somit ist ein Entspannen der Spannkomponente durch Betätigen des Spannhebels möglich. Beispielsweise wird der Spannhebel betätigt, indem der erste Arm gegen die Spannfeder gedrückt wird.

In einer Ausführungsform weist die Führungskomponente zwei voneinander beabstandete Führungselemente auf, die sich von dem Grundkörper in der gleichen Richtung erstrecken. Die beiden Führungselemente weisen je eine innere Seitenfläche, die der jeweils anderen der Führungselemente zugewandt ist, und eine der inneren Seitenfläche gegenüberliegende Außenfläche auf.

Die Führungselemente können somit zwei Führungen bereitstellen, eine auf den gegenüberliegenden inneren Seitenflächen sowie eine auf den äußeren Seitenflächen. Vorzugsweise sind die inneren Seitenflächen zur Führung des Spannzylinders und die äußeren Seitenflächen zur Führung einer Ablehr- und Bohrkomponente ausgelegt.

In einer Ausführungsform weist jede der inneren Seitenflächen eine konkave zylindrische Form auf, derart, dass die beiden inneren Seitenflächen mit dem dazwischenliegenden Raum eine zylindrische Führung definieren. Vorteilhafterweise wird dadurch eine zylindrische Führung insbesondere für den Spannzylinder definiert. Die Führung ermöglicht eine Drehbarkeit des Spannzylinders um die Zylinderachse. Vorzugsweise weist eine der Seitenwände der Führungselemente einen Durchbruch auf, durch den der Spannhebel von der unteren Seite des Grundkörpers reicht. Indem der Spannzylinder mit dem Rastelement auf der Seite eingesetzt ist, an dem der Durchbruch vorgesehen ist, kann das Spannelement in der Führung bezüglich des Spannhebels gespannt werden.

In einer Ausführungsform ist ein Abstand zwischen den inneren Seitenflächen auf einer vorderen Seite des Grundkörpers von einem Abstand zwischen den Seitenflächen auf einer hinteren Seite verschieden.

Zusätzlich kann ein Vorsprung auf dem Spannzylinder vorgesehen sein, der lediglich durch die größere der Öffnungen passt. Somit ist ein fehlerhaftes Einsetzen, beispielsweise durch 180° Drehung des Spannzylinders um seine Achse, nicht möglich. Mit anderen Worten ist ein Einsetzen des Spannzylinders nur in der richtigen Stellung möglich. In anderen Ausführungsformen kann der Spannzylinder auch derart ausgestaltet sein, dass er in zwei Stellungen bezüglich seiner Längsachse eingesetzt werden kann.

In einer Ausführungsform weist in Erstreckungsrichtung der Führungselemente eine der Außenflächen eine Vertiefung und die andere der Außenflächen einen Vorsprung zur Führung einer Ablehr- und Bohrkomponente auf. Indem eine der Flächen eine Vertiefung und die andere der Flächen einen Vorsprung aufweist, kann die Ablehr- und Bohrkomponente nicht um 180° bezüglich der Führungselemente verdreht aufgesetzt werden. Somit wird eine fehlerhafte Bedienung der Operationsvorrichtung vermieden. Die Führungselemente definieren somit auf ihren Außenflächen eine lineare Führung der Ablehr- und Bohrkomponente, die keine relative Rotation erlaubt.

In einer Ausführungsform erstrecken sich die Führungselemente in einem vorderen Bereich von dem Grundkörper und ein Radius des Grundkörpers entspricht der Tibia-Grundfläche. Vorzugsweise wird von einer durchschnittlichen Tibiafläche ausgegangen und die Grundfläche des Grundkörpers entspricht der durchschnittlichen Tibiafläche. Die Form der Tibiafläche ist bekannt. In anderen Ausführungsformen kann vorgesehen werden, zwei oder mehrere verschiedene Grundflächen für stark vom Durchschnitt abweichende Tibiagrößen vorzusehen.

In einer Ausführungsform läuft der Radius des Grundkörpers auf den Radius der Ablehr- und Bohrkomponente aus. Dadurch können insbesondere Verletzungs- oder Fehlhandhabungsgefahren, die durch Kanten oder hervorstehende Ecken bestehen, ausgeschlossen werden.

Indem der Lehrenaufsatz eine Nut für einen oberen Femurschnitt aufweist, kann mittels des Lehrenaufsatzes der obere Femurschnitt abgelehrt werden.

In einer Ausführungsform weist die Ablehr- und Bohrkomponente ein Befestigungsmittel auf, dass dazu eingerichtet ist, die Position des Lehrenaufsatzes bezüglich der Führungskomponente festzusetzen.

Wenn die Führungskomponente bezüglich der Spannkomponente zwischen Femur und Tibia gespannt ist, kann mittels der Ablehr- und Bohrkomponente die Größe der später einzusetzenden Prothese festgelegt werden. Wenn sich der Lehraufsatz bezüglich der Führungskomponente und damit bezüglich des Femur an der korrekten Stelle befindet, kann die Position mittels des Befestigungsmittels festgesetzt werden. Damit kann ein sicheres Ablehren des oberen Femurschnittes und ein sicheres Bohren der Prothesenbohrungen ermöglicht werden.

In einer Ausführungsform ist das Befestigungsmittel in Form einer Stellschraube ausgestaltet und der Lehrenaufsatz weist ein der Stellschraube entsprechendes Gewinde auf. Das der Stellschraube entsprechende Gewinde des Lehrenaufsatzes ist in einer Ausführungsform an eine seitliche Außenfläche des Lehrenaufsatzes angebracht und reicht durch den Lehrenaufsatz hindurch bis zu dem Führungselement. Damit kann durch ein Festschrauben der Stellschraube die Position des Lehrenaufsatzes bezüglich der Führungskomponente festgesetzt werden.

In einer Ausführungsform ist das Befestigungsmittel auf einer Vorderseite der Ablehr- und Bohrkomponente angeordnet. Die Vorderseite der Ablehr- und Bohrkomponente ist die Seite, die der Femurstirnfläche abgewandt, also dem Operateur zugewandt ist. Durch die Anordnung auf der Vorderseite ist ein Einsatz des Befestigungsmittel sowohl bei einer Operation des linken als auch des rechten Knies möglich, ohne dass seitlich der Ablehr- und Bohrkomponente liegende Ligamente die Anordnung des Befestigungsmittels behindern könnten.

In einer Ausführungsform ist das Befestigungsmittel in Form eines Absteckers ausgestaltet. Der Lehrenaufsatz weist wenigstens eine Durchgangsöffnung auf und ein Führungselement weist wenigstens eine zugehörige Aussparung auf. Der Abstecker kann durch die Durchgangsöffnung in die Aussparung eingesetzt werden und dadurch die Position des Lehrenaufsatzes bezüglich des Führungselementes festsetzen. Im Vergleich zu der Stellschraube ist mittels des Absteckers ein Festsetzen nur an vordefinierten Positionen des Führungselementes möglich.

In einer Ausführungsform sind die wenigstens eine Durchgangsöffnung und die wenigstens eine zugehörige Aussparung rechteckig ausgestaltet. Hierdurch kann ein sicherer Halt des Absteckers in der Durchgangsöffnung und der zugehörigen Aussparung erhalten werden, da der Abstecker gegen ein Verdrehen gesichert ist.

Vorzugsweise unterscheiden sich die Abstände der Durchgangsöffnungen von dem Grundkörper bezüglich der Abstände des Lehrenaufsatzes, sodass ein Festsetzen mittels des Absteckers in verschiedene Durchgangsöffnungen verschiedener Positionen des Lehrenaufsatzes bezüglich des Grundkörpers definiert.

Hiermit wird ermöglicht, dass eine bestimmte Orientierung eine bestimmte Position des Lehrenaufsatzes bezüglich des Führungselements definiert. Mit anderen Worten, eine fehlerhafte, beziehungsweise ungewollte Positionierung des Lehrenaufsatzes kann dadurch verhindert werden, dass einer bestimmten Position eine bestimmte Orientierung entspricht. Vorzugsweise weisen die voneinander verschiedenen Durchgangsöffnungen um jeweils 30°, besonders bevorzugt um jeweils mindestens 45° voneinander verschiedene Orientierungen auf.

In einer Ausführungsform weist der Lehrenaufsatz eine Bogenform auf und wird durch Außenflächen zweier Führungselemente geführt. Mit anderen Worten, der Lehrenaufsatz wird vorzugsweise seitlich auf die Führungselemente aufgeschoben. Die Führung durch die Außenflächen der beiden Führungselemente unterscheidet sich hierbei vorzugsweise zwischen den Führungselementen, indem beispielsweise auf einer Außenfläche eine Nut und auf der anderen ein Vorsprung bereitgestellt wird. Somit kann verhindert werden, dass der Lehrenaufsatz in der falschen Orientierung aufgesetzt wird. Vorzugsweise ist mindestens eine, insbesondere sind beide, der auf den Außenflächen definierten Führungen als Schwalbenschwanzführungen ausgeführt.

In einer Ausführungsform weist der Lehrenaufsatz wenigstens eine Nut für das Ablehren eines unteren Femurschnittes auf. Indem der Lehrenaufsatz eine Nut für einen unteren Femurschnitt aufweist, muss keine weitere Lehrenvorrichtung für das Ablehren der unteren Femurseite vorgesehen werden. Dies reduziert den Aufwand während der Operation und vermeidet, dass der entfallene, zusätzliche Lehrenaufsatz fehlerhaft angebracht wird.

In einer Ausführungsform sind die Schneideebenen für den oberen Femurschnitt und den unteren Femurschnitt nicht parallel. Insbesondere schließt die obere Schnittebene einen Schnittwinkel von 95° mit der Femurstirnfläche ein. Die Orientierungen der Schneidebenen für den oberen Femurschnitt und den unteren Femurschnitt sind dabei insbesondere nach den Anforderungen der Prothese ausgelegt. In anderen Ausführungsformen können die Schneidebenen demnach auch andere Winkel mit der Femurstirnfläche einschließen. Ferner können in weiteren Ausführungsformen beispielsweise auch weitere Schnittebenen, wie beispielsweise diagonale Schnittebenen, durch den Lehrenaufsatz ermöglicht werden. Dies ermöglicht, dass der erfindungsgemäße Lehrenaufsatz mit mehreren Prothesen kompatibel ist, wodurch sich die Einsetzbarkeit der erfindungsgemäßen Operationsvorrichtung verbessert.

In einer Ausführungsform weist der Lehrenaufsatz zwei Bohrbuchsen zum Ausführen der Femur-Prothesenbohrungen auf. Vorzugsweise sind die Bohrbuchsen symmetrisch und in jeweils einem Arm des bogenförmigen Lehrenaufsatzes angeordnet. Die Position der Bohrbuchsen entspricht der finalen Bohrposition des Femur-Prothesenelementes. In einer Ausführungsform sind die Bohrbuchsen integral in dem Lehrenaufsatz integriert. In dieser Ausführungsform weist der Lehrenaufsatz somit ein Material hinreichender Härte auf. In einer anderen Ausführungsform sind die Bohrbuchsen als Bohrbuchseneinsatz ausgeführt, insbesondere somit in den Lehrenaufsatz geklemmt oder eingepresst.

In einer Ausführungsform weist der Lehrenaufsatz eine Millimeterskala auf seiner Vorderseite auf. Die Millimeterskala wird durch eine Kennzeichnung auf der Führungskomponente referenziert. Die Millimeterskala ermöglicht es einem Operateur, die Operationsvorrichtung beziehungsweise insbesondere den Lehrenaufsatz genau und in nicht mittels des Absteckers absteckbaren Zwischenabständen zu positionieren.

Ferner wird eine Verwendung einer erfindungsgemäßen Operationsvorrichtung zur Operation eines menschlichen Knies, insbesondere bei einer Implantation einer Knie-Endoprothese, offenbart.

Weitere Ausgestaltungen, Ausführungsformen sowie damit verbundene Vorteile werden im Folgenden mit Bezug auf die beigefügten Figuren beschrieben.

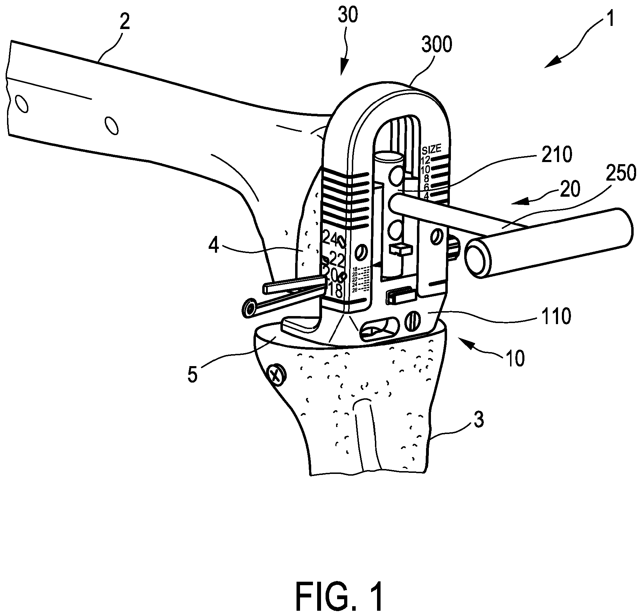

- Fig. 1: zeigt eine schematische perspektivische Ansicht einer Operationsvorrichtung gemäß einem Ausführungsbeispiel der vorliegenden Erfindung,

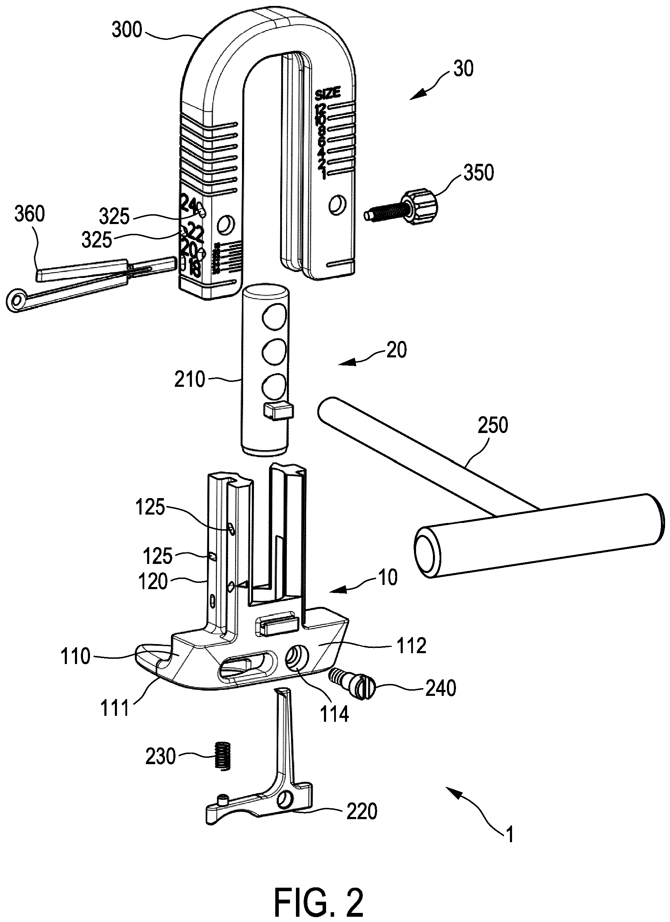

- Fig. 2: zeigt eine schematische beispielhafte Explosionsansicht der Operationsvorrichtung gemäß einem Ausführungsbeispiel der Erfindung,

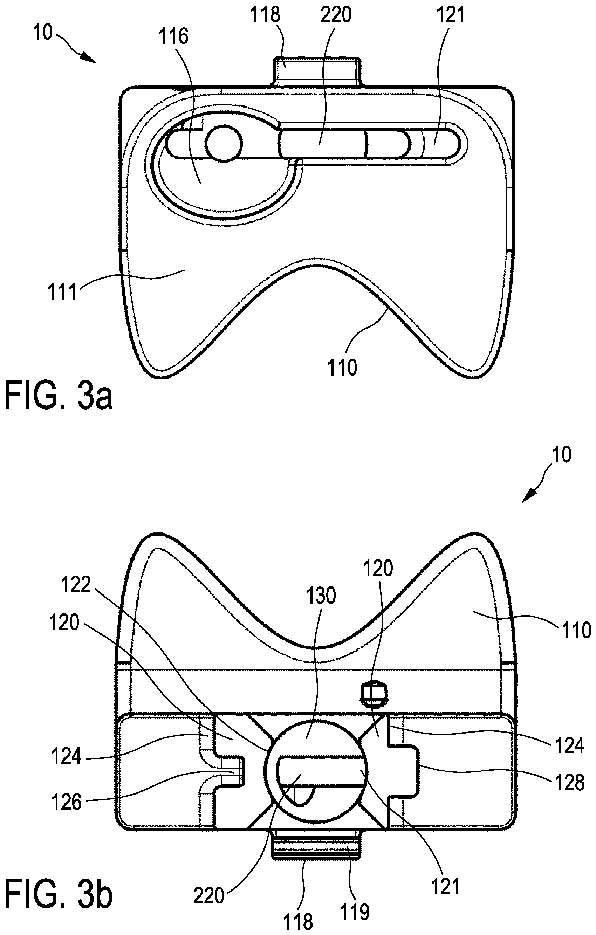

- Fig. 3a - 3e: zeigen schematisch und exemplarisch eine Führungskomponente einer Operationsvorrichtung gemäß einem Ausführungsbeispiel in verschiedenen Ansichten,

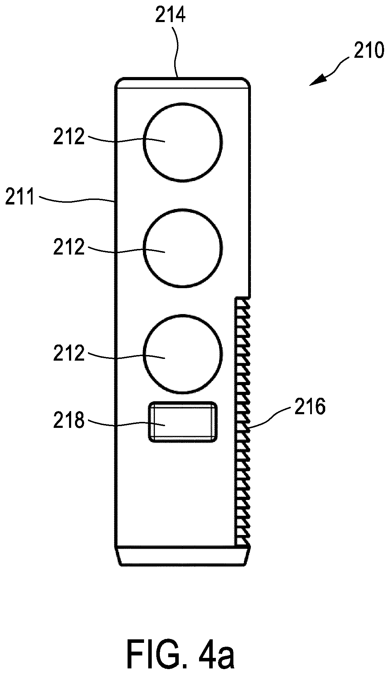

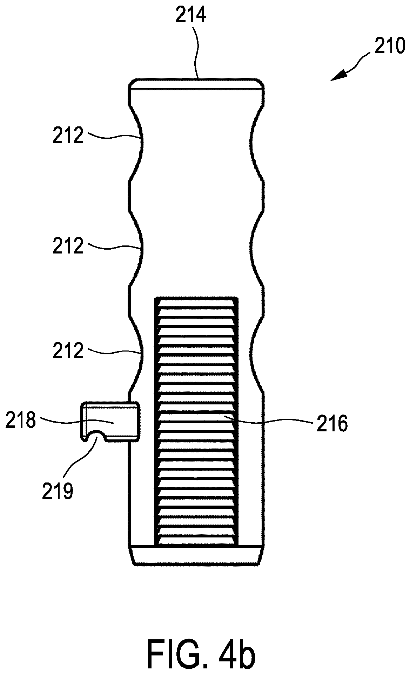

- Fig. 4a und 4b: zeigen schematisch und exemplarisch zwei Ansichten eines Spannzylinders gemäß einem Ausführungsbeispiel,

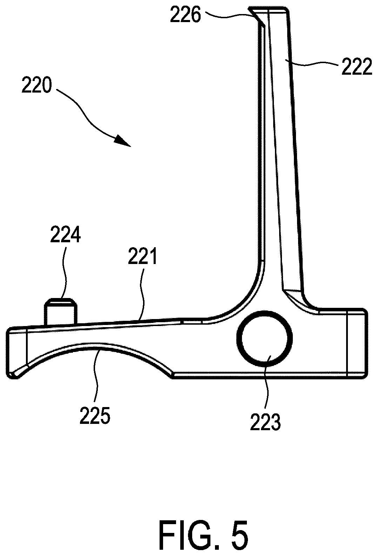

- Fig. 5: zeigt schematisch und exemplarisch einen Spannhebel gemäß einem Ausführungsbeispiel,

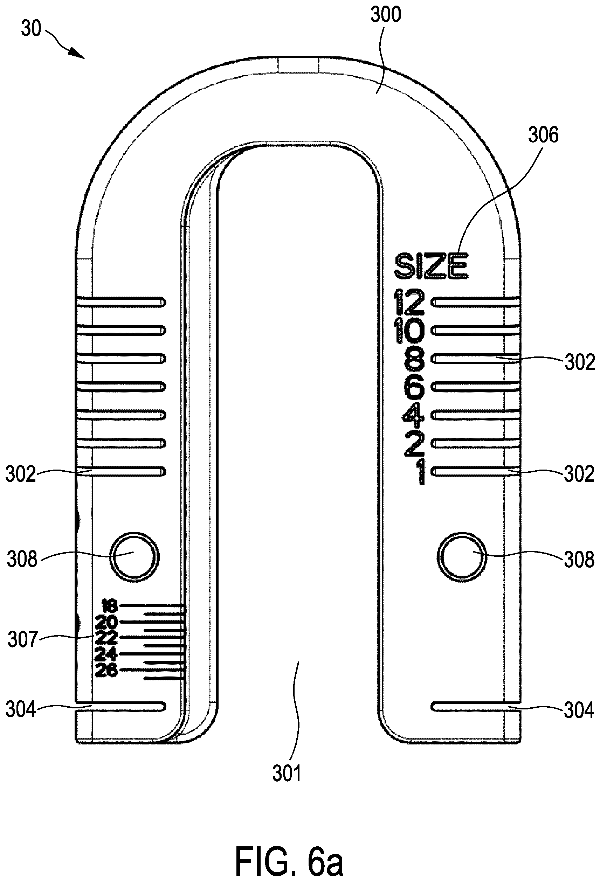

- Fig. 6a - 6d: zeigen schematisch und exemplarisch vier Ansichten eines Lehrenaufsatzes gemäß einem Ausführungsbeispiel,

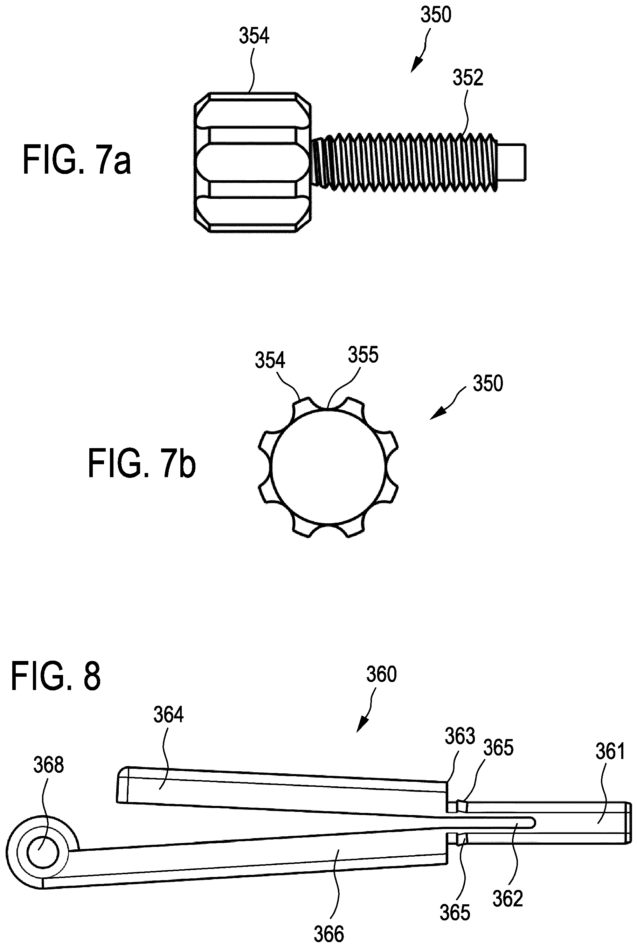

- Fig. 7a und 7b: zeigen schematisch und exemplarisch zwei Ansichten einer Stellschraube gemäß einem Ausführungsbeispiel,
- Fig. 8: zeigt schematisch und exemplarisch einen Abstecker gemäß einem Ausführungsbeispiel,

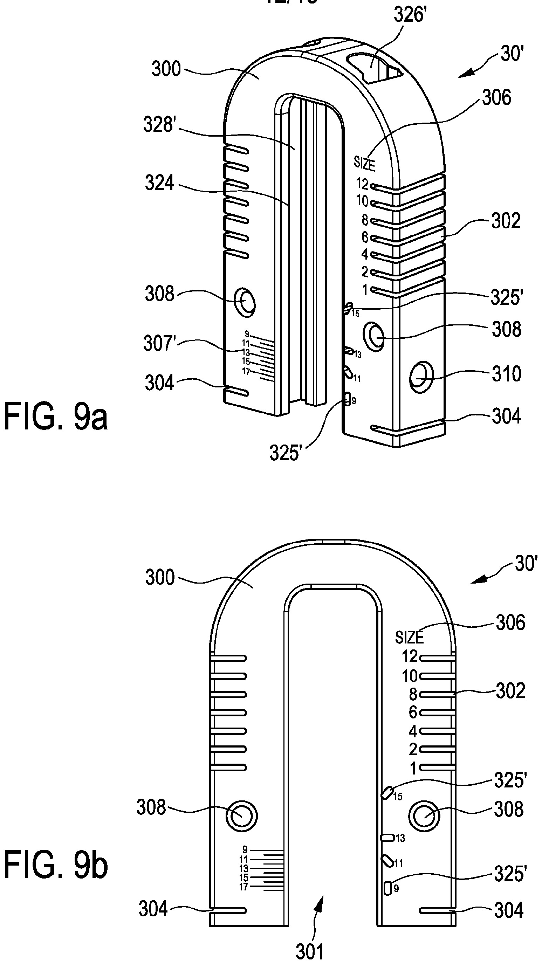

- Fig. 9a und 9b: zeigen schematisch und exemplarisch zwei Ansichten eines weiteren Ausführungsbeispiels eines Lehrenaufsatzes und

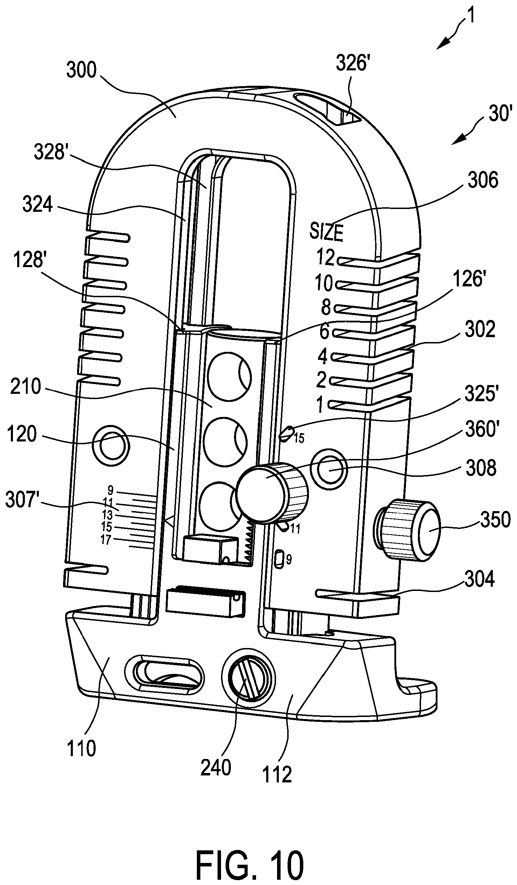

- Fig. 10: zeigt schematisch und exemplarisch eine perspektivische Ansicht einer Operationsvorrichtung mit dem Lehrenaufsatz der Fig. 9.

Fig. 1 zeigt ein Ausführungsbeispiel einer Operationsvorrichtung 1, wie sie während einer Knieoperation in einem Kniegelenk zum Einsatz kommt. Fig. 1 zeigt schematisch ein Femur 2 sowie eine Tibia 3, die in einem abgewinkelten Zustand angeordnet sind. Vor Einsatz der erfindungsgemäßen Operationsvorrichtung 1 sind sowohl einen Femurstirnfläche 4 sowie eine Tibiastirnfläche 5 für die Prothesenoperation vorbereitet worden. Fig. 1 zeigt schematisch lediglich Femur 2 und Tibia 3, alle weiteren Elemente des Kniegelenks sind zur besseren Darstellbarkeit weggelassen.

Die Operationsvorrichtung 1 weist eine Führungskomponente 10, eine Spannkomponente 20 sowie eine Ablehr- und Bohrkomponente 30 auf. Durch die Spannkomponente 20 wird die Operationsvorrichtung 1 derart zwischen Femur und Tibia gespannt, dass die Ligamente (nicht gezeigt) gleichmäßig gespannt sind. Dadurch werden eine Fehlstellung der Prothese und eine darauffolgende Fehlstellung des Beines vermieden.

Die Führungskomponente 10 weist einen Grundkörper 110 auf, der auf der Tibiagrundfläche 5 aufliegt. Die Spannkomponente 20 umfasst einen Spannzylinder 210, der die Führungskomponente 10 gegen die Spannkomponente 20 spannt. Der Spannzylinder 210 ist mittels eines T-Halters 250 an der Femurstirnfläche 4 fixiert. Dadurch kann ein ungewolltes Verschieben des Spannzylinders 210 bezüglich der Femurgrundfläche 4 verhindert werden.

Die Ablehr- und Bohrkomponente 30 weist einen Lehrenaufsatz 300 auf, der auf die Führungskomponente 10 aufsteckbar ist. Wie in Fig. 1 ersichtlich, kann der Lehrenaufsatz 300 auf die Operationsvorrichtung 1 aufgesteckt sein und somit ein Ablehren und Bohren des Femur 2 ermöglichen, ohne dass die Spannkomponente 20 entspannt und von dem Gelenk entfernt werden muss.

Fig. 2 zeigt die einzelnen Komponenten der Operationsvorrichtung 1 in einer schematischen Explosionsdarstellung. Die Führungskomponente 10 weist einen Grundkörper 110 und zwei parallele Führungselemente 120 auf, die sich von einer Grundfläche 111 des Grundkörpers 110 nach oben erstrecken. Die Grundfläche 111 ist dazu eingerichtet, auf der Tibiastirnfläche aufzuliegen. Die Führungselemente 120 sind dazu eingerichtet, zwei Komponenten zu führen, nämlich auf der Innenseite der Führungselemente 120 den Spannzylinder 210 sowie auf der Außenseite der Führungselemente 120 die Ablehr- und Bohrkomponente 30, insbesondere den Lehrenaufsatz 300.

Der Grundkörper 110 weist verschiedene Öffnungen auf, die dazu eingerichtet sind, verschiedene andere Komponenten der Operationsvorrichtung 1 aufzunehmen. Auf der Grundfläche 111 weist der Grundkörper 110 eine Öffnung für die Aufnahme einer Spannfeder 230 auf sowie eine weitere Öffnung, durch die ein Spannhebel 220 durch den Grundkörper 110 hindurch bis in die Führungselemente 120 eingeführt werden kann. Durch eine Befestigungsöffnung 114, die auf einer Vorderfläche 112 des Grundkörpers 110 vorgesehen ist, kann eine Befestigungsschraube 240 eingeführt werden, mittels derer der Spannhebel 220 drehbar an der Führungskomponente 10 befestigt wird.

Die Ablehr- und Bohrkomponente 30 weist den Lehrenaufsatz 300 auf, der mittels einer Stellschraube 350 und/oder eines Absteckers 360 an einer gewünschten Position bezüglich der Führungselemente 120 festgesetzt werden kann. Der Abstecker 360 wird durch Öffnungen 325 in dem Lehrenaufsatz 300 eingeführt und in korrespondierenden Öffnungen 125 auf den Außenflächen der Führungselemente 120 eingesetzt, beispielsweise eingerastet.

Die einzelnen Komponenten und Elemente der Operationsvorrichtung 1 werden im Folgenden im Detail und separat mit Verweis auf die weiteren Figuren beschrieben.

Figuren 3a bis 3e zeigen verschiedenen Ansichten der Führungskomponente 10. Fig. 3a zeigt eine Ansicht von unten, das heißt mit Sicht auf die Grundfläche 111 des Grundkörpers 110. Der Radius der Grundfläche 111 ist an die Tibiastirnfläche 5 angepasst und läuft mit konstant abnehmendem Radius auf den des Lehrenaufsatzes 300 aus. Dadurch können eventuelle Verletzungs- und Fehlhandhabungsgefahren ausgeschlossen werden. Fig. 3a zeigt eine Öffnung 116, in die der Spannhebel 220 eingesetzt ist. Der Spannhebel 220 führt durch eine weitere Aussparung 121 durch den Grundkörper 110 hindurch bis in die Führungselemente 120.

Der Grundkörper 110 zeigt auf einer Vorderseite eine Nase 118, die auf ihrer Oberseite eine Nut 119 (vgl. z.B. Fig. 3b) aufweist. Nase 118 und Nut 119 bilden einen Teil des tatsächlichen Spannmechanismus. Zwischen Nase 118 und einer korrespondierenden Nase 218 auf dem Spannzylinder 210 (vgl. Fig. 4) wird zum Spannen eine Zange (nicht gezeigt) eingesetzt. Über die Öffnung der Zange wird der Abstand zwischen Grundkörper 110 und Spannzylinder 210 bis zum gewünschten Grad der Spannung vergrößert. Damit die Zange nicht aus der Nase 118 oder der Nase 218 herausrutscht, weisen die jeweiligen Nasen 118, 218 auf den einander zugewandten Seiten korrespondiere Nuten 119, 219 auf.

In Fig. 3b ist die Führungskomponente 10 in einer Ansicht von oben gezeigt. Eine zylindrische Führungsaufnahme 130 ist zwischen den zwei Führungselementen 120 definiert. Hierfür definieren jeweilige Innenflächen 122 der Führungselemente 120 Abschnitte einer zylindrischen Führung. In dieser Ansicht ist auch die Aussparung 121 sichtbar, durch die der Spannhebel 220 in die zylindrische Führungsaufnahme 130 anliegt. Fig. 3b zeigt weiter Außenflächen 124 der Führungselemente 120, die eine zweite Führung für die Ablehr- und Bohrkomponente 30 bilden. Um ein fehlerhaftes Aufsetzen der Ablehr- und Bohrkomponente 30 auf die Führungselemente 120 zu vermeiden, ist auf der in der Zeichnung links gezeigten Seitenfläche 124 ein Führungsvorsprung 128 ausgebildet, wohingegen auf der in der Zeichnung rechts gezeigten Seitenfläche 124 eine Führungsnut 126 ausgebildet ist.

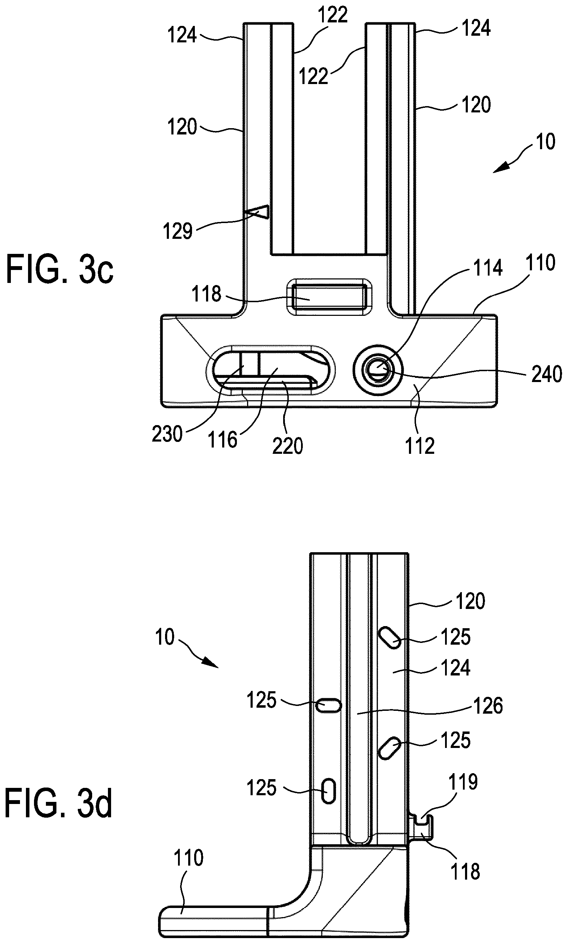

Fig. 3c zeigt die Führungskomponente 10 von vorne. Die Vorderfläche 112 weist die Befestigungsöffnung 114 auf, in die die Befestigungsschraube 240 eingesetzt ist. Ferner ist die Öffnung 116 erkennbar, die einen Zugriff auf den Spannhebel 220 ermöglicht. Über die Öffnung 116 kann der Spannhebel 220 gegen die Spannfeder 230 gedrückt werden und somit eine Spannung des Spannhebels gelöst werden. Auf der Vorderseite der Führungselemente 120 ist ein Indikator 129 gezeigt. Der Indikator 129 kann dazu verwendet werden, eine Größenangabe, die an entsprechender Stelle auf der Ablehr- und Bohrkomponente 30 bereitgestellt ist, abzulesen.

Fig. 3d zeigt eine Seitenansicht von links auf die Führungskomponente 10. Die Führungsnut 126 verläuft entlang der Seitenfläche 124 in der Erstreckungsrichtung der Führungselemente 120, insbesondere in der Mitte der Seitenfläche 124. In der Seitenansicht ist ferner die Nase 118 mit der Nut 119 auf deren Oberseite gezeigt, mittels der die Spannung der Führungskomponente 10 gegen die Spannkomponente 20 erfolgt. Je zwei Öffnungen 125 sind auf jeder Seite der Seitenfläche 124 links und rechts der Führungsnut 126 bereitgestellt. Die Öffnungen 125 haben verschiedene Ausrichtungen, um ein fälschliches Abstecken mittels des Absteckers 360 zu verhindern. Die Ausrichtungen der Geometrie der Öffnungen 125 sind nach der bekannten Methode "Poka Yoke" gestaltet.

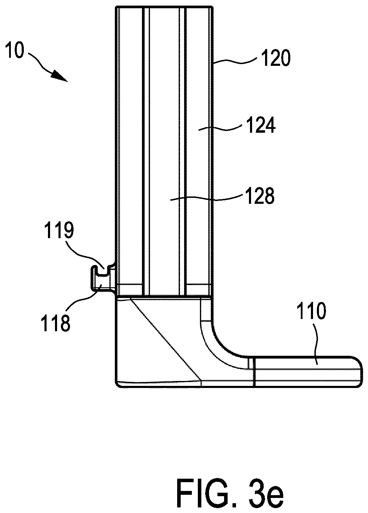

Schließlich zeigt Fig. 3e eine Seitenansicht von rechts auf die Führungskomponente 10. Der Führungsvorsprung 128 der Seitenfläche 124 verläuft entlang der Seitenfläche 124 in der Erstreckungsrichtung der Führungselemente 120.

Fig. 4a zeigt eine Vorderansicht auf einen beispielhaften Spannzylinder 210 und Fig. 4b zeigt eine beispielhafte Seitenansicht auf den Spannzylinder 210. Der Spannzylinder 210 besteht im Wesentlichen aus einem zylindrischen Stift 211. Quer zur Längsrichtung des zylindrischen Stifts 211 sind in diesem Beispiel drei Durchgangsbohrungen 212 vorgesehen. Die Durchgangsbohrungen 212 sind jeweils parallel zueinander durch die Mitte des zylindrischen Stifts 211 verlaufend. Mittels der verschiedenen Durchgangsbohrungen 212 sind unterschiedliche Größeneinstellungen der Operationsvorrichtung 1 möglich. In diesem Beispiel ist die mittlere der Durchgangsbohrungen 212 für eine Standardgröße vorgesehen. Im Falle eines besonders großen, beziehungsweise besonders kleinen Knies kann die obere oder untere Durchgangsbohrung 212 zur Fixierung der Operationsvorrichtung 1 mittels des T-Halters 250 an das Femur verwendet werden. In anderen Beispielen kann der Spannzylinder 210 auch nur eine oder mehr als drei Durchgangsbohrungen aufweisen.

Der Spannzylinder 210 weist weiter eine Aussparung in Längsrichtung 214 auf. Vorzugsweise reicht die Aussparung 214 vollständig durch den zylindrischen Stift 211 durch. Der zylindrische Stift 211 kann somit auch als Hohlzylinder beschrieben werden. Die Aussparung 214 kann allerdings auch nur einen Teil der Länge des zylindrischen Stifts 211 reichen. Insbesondere ist die Aussparung 214 dafür eingerichtet, einer Verklemmung aufgrund von Körpergeweben beziehungsweise Körperflüssigkeiten vorzubeugen.

Auf der in Fig. 4a im Bild rechts gezeigten Mantelfläche weist der zylindrische Stift 211 ein Rastprofil 216 auf. Das Rastprofil 216 ist derart eingerichtet, dass es mit einem korrespondierenden Rastprofil des Spannhebels 220 einrastet, um eine Spannung des Spannzylinders 210 bezüglich des Spannhebels und der Führungskomponente 10 zu ermöglichen. In diesem Ausführungsbeispiel ermöglicht das Rastprofil 216 eine Entfernung des Spannhebels 220 und des Spannzylinders 210 voneinander, während eine Bewegung der jeweiligen Elemente aufeinander zu blockiert ist. In anderen Ausführungsformen können auch andere Rastprofile oder andere geeignete Mechanismen vorgesehen werden, die ein Spannen des Spannzylinders 210 und des Spannhebels 220 ermöglichen. Zum Spannen weist der Spannzylinder 210 ferner auf seiner Vorderseite die Nase 218 auf, die zum Spannen mittels einer nicht gezeigten Zange gegenüber der korrespondierenden Nase 118 eingerichtet ist. Die Nase 218 stellt somit einen Zangenansatz dar.

Fig. 5 zeigt eine Seitenansicht auf den beispielhaften Spannhebel 220. Der Spannhebel 220 dient als ein Spann- und Lösehebel und weist einen ersten Hebelarm 221 und einen zweiten Hebelarm 222 auf. Der Spannhebel 220 ist drehbar bezüglich eines Drehpunktes 223 ausgelegt. An dem Drehpunkt 223 wird der Spannhebel 220 mittels der Befestigungsschraube 240 an der Führungskomponente 10 befestigt. Eine Bewegung des ersten Hebelarms 221 führt somit zu einer entsprechenden Drehbewegung des zweiten Hebelarms 222. Der Spannhebel 220 weist ferner an seinem ersten Hebelarm 221 einen hervorstehenden Zylinder 224 sowie eine gebogene Aussparung 225 auf. Der Zylinder 224 ist zur Sicherung der Spannfeder 230 ausgestaltet, die Aussparung 225 liegt dem Zylinder 224 gegenüber und ermöglicht, dass über die Öffnung 116 des Grundkörpers 110 der erste Hebelarm 221 in Richtung des Zylinders 224 gegen die Kraft der Spannfeder 230 bewegt wird. Durch die Bewegung des ersten Hebelarms 221 in der Zeichnung nach oben bewegt sich der zweite Hebelarm 222 in der Zeichnung nach rechts. Ein Rastelement 226, das an einem Ende des zweiten Hebelarms 222 vorgesehen ist, bewegt sich somit ebenfalls nach rechts. Durch die Vorspannung der Spannfeder 230 ist das Rastelement 226 gegen das Rastelement 216 des Spannzylinders 210 vorgespannt. Durch ein Drücken entgegen der Spannfeder 230 wird das Rastelement 226 aus der Vorspannung entfernt und ein Lösen des Spannzylinders 210 ist möglich. In der Seitenansicht ist eine Nut 219 auf der Unterseite der Nase 218 gezeigt, die ein Abrutschen einer zum Spannen eingesetzten Zange verhindert.

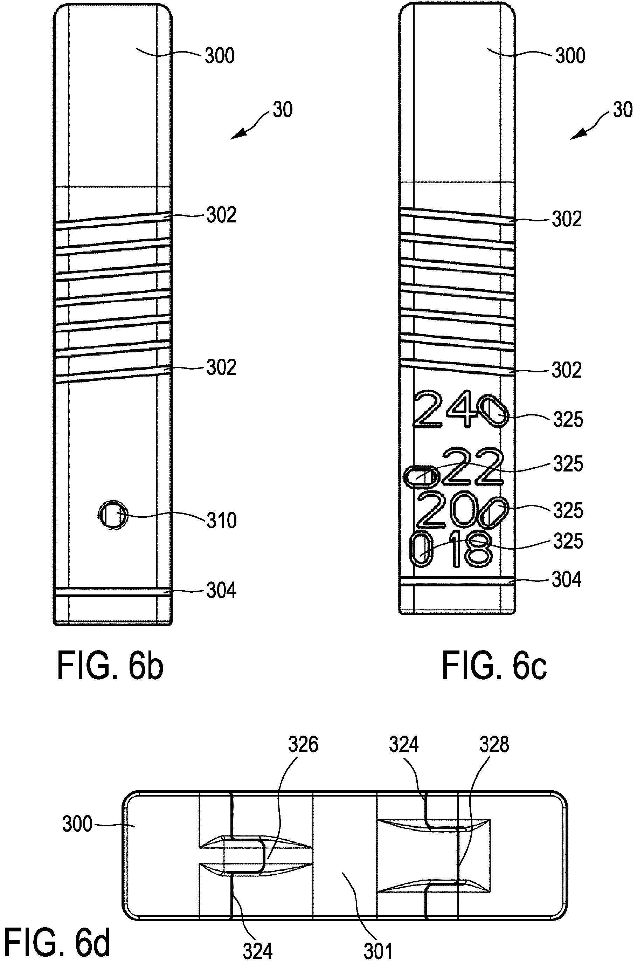

Fig. 6a zeigt eine Ansicht auf eine beispielhafte Ablehr- und Bohrkomponente 30 von vorne und Fig. 6b zeigt eine weitere Ansicht auf die beispielhafte Ablehr- und Bohrkomponente 30 von der Seite. Gezeigt ist ein Lehrenaufsatz 300, der auf die Führungselemente 120 aufgesetzt und durch die äußeren Seitenflächen 124 geführt wird. Der Lehrenaufsatz 300 ist in Form eines umgekehrten U ausgestaltet, sodass der Lehrenaufsatz 300 aufgrund eines ausgesparten Bereiches 301 auf die Führungskomponente 10 aufgesetzt werden kann. Der Lehrenaufsatz 300 weist im oberen Bereich mehrere Nuten 302 auf, die dazu eingerichtet sind, die obere Femurfläche in der gewünschten Prothesengröße abzulehren. Eine auf der Vorderseite aufgedruckte Größenangabe 306 definiert, für welche Prothesengröße welche Nut 302 zu verwenden ist. In diesem Beispiel sind die Standardgrößen 1, 2, 4, 6, 8, 10 und 12 angezeigt.

In anderen Beispielen des Lehrenaufsatzes 300 sind selbstverständlich auch andere Arten und Kombinationen von Nuten 302 und Größenangaben 306 vorstellbar. Der Lehrenaufsatz 300 weist im unteren Bereich ferner je eine untere Nut 304 auf, die dazu eingerichtet ist, auch den unteren Femurschnitt zu lehren. Zur Befestigung des Lehrenaufsatzes in dem Femur und der Festlegung der endgültigen Bohrposition beziehungsweise der Bohrung der endgültigen Prothesenbohrlöcher weist der Lehrenaufsatz 300 ferner je eine Bohrbuchse 308 in jedem Arm des Lehrenaufsatzes 300 auf. Die Bohrbuchse 308 ist in dem Beispiel integral mit dem Lehrenaufsatz 300 gebildet. Der Lehrenaufsatz 300 weist damit ein Material hinreichender Härte auf. In anderen Beispielen kann die Bohrbuchse 308 auch in bekannter Weise, beispielsweise geklemmt, in den Lehrenaufsatz 300 eingesetzt werden.

In der in Fig. 6b gezeigten Seitenansicht ist weiter ein Stellgewinde 310 gezeigt. Mittels des Stellgewindes 310 kann die Stellschraube 350 derart eingeschraubt werden, dass eine Fixierung zwischen Lehrenaufsatz 300 und Führungselementen 120 entsteht. Damit kann die Position der Ablehr- und Bohrkomponente 30 bezüglich der Führungskomponente 10 festgesetzt werden. In der in Fig. 6b gezeigten Seitenansicht ist ersichtlich, dass die oberen Nuten 302 nicht parallel zu den unteren Nuten 304 verlaufen. Insbesondere ist in dem Beispiel der Winkel zwischen Femurstirnfläche und oberen Nuten 302 95°. In weiteren Beispielen des Lehrenaufsatzes 300 können auch zusätzliche Nuten, beispielsweise zur Durchführung diagonaler Schnitte, vorgesehen sein und die Winkel der verschiedenen Nuten können unterschiedlich sein.

Fig. 6c zeigt eine weitere Seitenansicht des Lehrenaufsatzes 300, in der die Öffnungen 325 für den Abstecker 260 gezeigt sind, die in verschiedenen Höhen über der Führungskomponente 10 Positionierungen des Lehrenaufsatzes 300 ermöglichen. Neben jeder der Öffnungen 325 zeigt eine Zahl eine mit der Öffnung in Beziehung stehende Größe, beispielsweise eine Größe der Prothese oder eine Längenangabe in Millimetern oder ähnliches, an.

Fig. 6d zeigt eine Ansicht von unten auf den Lehrenaufsatz 300. Gegenüberliegende innere Seitenwände 324 sind dazu eingerichtet, mit korrespondierenden Seitenwänden 124 der Führungskomponente 10 zusammenzupassen. Zwischen den Seitenwänden 324 ist der ausgesparte Bereich 301 angedeutet, in dem die Führungselemente 120 aufgenommen werden. Ein Vorsprung 326 auf der in der Figur links gezeigten Seitenwand 324 ist dazu eingerichtet, in die Führungsnut 126 eingesetzt zu werden und darin geführt zu werden. Eine Nut oder Aussparung 328 auf der gegenüberliegenden Seitenfläche 328 ist dazu eingerichtet, mit dem Führungsvorsprung 128 des Führungselementes 120 zusammenzuwirken.

Fig. 7a zeigt eine Seitenansicht auf ein Ausführungsbeispiel der Stellschraube 350, und Fig. 7b zeigt eine Draufsicht auf die Stellschraube 350 von Seiten eines Schraubenkopfes 354. Die Stellschraube 350 weist ein Gewinde 352 sowie an einem Ende den Schraubenkopf 354 auf. Der Schraubenkopf 354 ist derart ausgestaltet, dass er durch Finger besonders gut betätigbar ist. Hierfür ist der Umlauf des Schraubenkopfes 354 nicht glatt, sondern weist regelmäßige Täler 355 auf. In anderen Ausführungsbeispielen sind selbstverständlich auch andere Schrauben einsetzbar. Vorzugsweise sind auch alternative Schrauben beziehungsweise Befestigungselemente ausgestaltet, durch manuelle Betätigung und nicht durch Betätigung mit Werkzeugen festgestellt werden zu können. Beispielsweise eignet sich hierzu eine Längsrändelung.

Fig. 8 zeigt beispielhaft eine Seitenansicht eines Absteckers 360, der eine weitere Möglicheit darstellt, wie die Ablehr- und Bohrkomponente 30 bezüglich der Führungskomponente 10 festgesetzt werden kann. Ein erstes Ende 361 des Absteckers 360 wird durch eine Abstecköffnung 325 des Lehrenaufsatzes 300 hindurch und in eine Abstecköffnung 125 auf einer äußeren Seitenfläche des Führungselementes 120 eingesteckt. Von dem ersten Ende 361 weg spaltet sich der Abstecker 360 an einer Gabelung 362 auf und verläuft in einem ersten Arm 364 und einem zweiten Arm 366 weiter. Der Abstecker wird bis zu einer Kante 363 in die Abstecköffnung 325 eingeführt. Der Abstand zwischen dem ersten Arm 364 und dem zweiten Arm 366 im Bereich der Kante 363 ist bereits größer als der Durchmesser der Abstecköffnung 325, sodass der Abstecker 360 durch die Biegespannung zwischen den zwei Armen 364, 366 in der Abstecköffnung 325 gehalten wird. Sowohl auf dem ersten Arm 364 als auch auf dem zweiten Arm 366 kann in dem Bereich, der in die Abstecköffnung 325 eingeführt wird, ein Vorsprung 365 ausgestaltet sein, der ein ungewolltes Lösen und Entfernen des Absteckers 360 aus der Abstecköffnung 325 verhindert. An einem dem ersten Ende 361 gegenüberliegenden Ende ist ferner eine Öse 368 bereitgestellt. Bei Bedarf kann an die Öse 368 eine Kette angebracht werden und dadurch ein Verlust des Absteckers im offenen Knie ausgeschlossen werden. Dadurch wird die Operationssicherheit weiter verbessert.

Fig. 9a und Fig. 9b zeigen schematisch und exemplarisch zwei Ansichten einer weiteren Ausführung eines Lehrenaufsatzes 30'. Der Lehrenaufsatz 30' übernimmt wesentliche Elemente des Lehrenaufsatzes 30, der beispielsweise in Figuren 6a bis 6d gezeigt ist. Gleiche Bezugsziffern beziehen sich auf gleiche Elemente und werden im Folgenden nicht erneut beschrieben.

Der Lehrenaufsatz 300 weist Öffnungen 325' auf seiner Vorderseite auf. Die Öffnungen 325' erfüllen die gleiche Funktionalität wie die Öffnungen 325, sind jedoch auf der Vorderseite angeordnet, um eine Verwendbarkeit bei Operationen sowohl des linken Knies als auch des rechten Knies besser gewährleisten zu können. In die Öffnungen 325' ist beispielsweise zur Fixierung der Ablehr- und Bohrkomponente 30' ein Abstecker 360 einsetzbar.

Weiter unterscheiden sich die Führungen 326' und 328' auf der inneren Seitenwand 324 des Lehrenaufsatzes 300. In dem in Fig. 9 gezeigten Beispiel sind beide der Führungen 326' und 328' als Schwalbenschwanzführungen ausgestaltet.

Schließlich unterscheidet sich die Beschriftung 307', wobei in der Fig. 9 anstelle der beispielsweise in Fig. 6 gezeigten Angaben in Millimetern nun eine sogenannte PE-size, also eine Größe der Prothese gezeigt ist. In anderen Ausführungen sind selbstverständlich auch andere oder überhaupt keine Beschriftungen möglich.

Fig. 10 zeigt die in Fig. 9 gezeigte Ablehr- und Bohrkomponente 30' in einer Position auf die Führungselemente 120 der erfindungsgemäßen Operationsvorrichtung 1 aufgeschoben und fixiert. In diesem Beispiel fixiert ein Abstecker 360' die Öffnung 325', die zu einer Prothese der Größe 13 gehört. Der Abstecker 360' weist abweichend von dem in der Fig. 8 gezeigten einen runden Kopf mit einer darin ausgeführten Querbohrung anstelle der Öse 368 auf. Um den runden Kopf weist der Abstecker 360' eine Längsrändelung auf. Zu den Führungen 326' und 328' korrespondierend weisen die Führungselemente die Gegenstücke der Schwalbenschwanzführungen 126' und 128' auf.

Im Folgenden werden weitere Vorteile und Verbesserungen des Ausführungsbeispiels der erfindungsgemäßen Vorrichtung gegenüber dem Stand der Technik ausgeführt.

Durch die erfindungsgemäße Vorrichtung werden mehrere operativ voneinander unabhängige Funktionen in einer Operationsvorrichtung 1 kombiniert. Eine gleichmäßige Spannung der Ligamente kann erzielt werden, damit einhergehend verläuft eine korrekte Rotation/Ausrichtung des Femur sowie eine reproduzierbare Ermittlung der individuell benötigten Prothesengröße in einer kombinierten Operationsvorrichtung 1. Die Operationsvorrichtung 1 ermöglicht in der Beugestellung während der Operation ein Ablehren der oberen und der unteren Schnittlage des Femur sowie ein Bohren der zwei Befestigungsbohrungen für die Prothesenbefestigung in der Femurstirnfläche 4.

Durch die erfindungsgemäße Operationsvorrichtung 1 wird eine Winkelbeweglichkeit des Spannzylinders 210 um die Rotationsachse des Spannzylinders 210 erhöht. Mit einer eingebauten Kraftübertragungseinrichtung, nämlich dem T-Halter 250, und durch eine Abänderung der Führung des Spannzylinders 210 gegenüber dem Stand der Technik wird der Rotationswinkel des Spannzylinders 210 erhöht.

Die Spannvorrichtungsmechanik, die eine Spannung des Spannhebels 220 und des Spannzylinders 210 implementiert, ermöglicht eine komplette Demontage des Spannhebels 220. Damit wird die Desinfizierbarkeit erhöht und eine Ablagerung von Körperflüssigkeiten und/oder Geweberesten vermieden. Die Anordnung und Geometrie der Führungskomponente und der Spannkomponente 20 ermöglicht einer Verlagerung beweglicher Teile in die Führungskomponente 10, wodurch eine unbeabsichtigte Mechanismusbetätigung, beispielsweise des Spannhebels 220, durch abstehende Bedienelemente vermieden wird.

Der Spannzylinder 210 ist dadurch optimiert, dass in Längsrichtung eine Aussparung 214 zur Aufnahme von etwaigen Fremdkörpern, wie Gewebe, Knochen, Knorpel oder ähnlichem, im Spannungszustand vorgesehen ist, und dahingehend eine Vermeidung einer Verklemmung der Spannkomponente 20 durch Materialquetschung/Anhäufung erreicht wird.

Die erfindungsgemäße Operationsvorrichtung 1 kombiniert mehrere Lehr- beziehungsweise Bohrvorrichtungen, beispielsweise eine Lehre für Femurober- und -unterschnitt sowie eine Lehre und Bohrschablone für Femurbohrungen zur Vereinfachung des Operationsprozesses, zur Vereinfachung der Operationsabfolgen, zur Verkürzung der Operationszeit, zur Optimierung der Operationsqualität, durch mechanisch vorbestimmte Prozessabfolgen, die durch einen aufsteckbaren Lehrenaufsatz 300 sowie Nuten 302 der Lehre für Femuroberschnitt realisiert werden, die durch Zahlenwerte zur Größenbestimmung der Prothese gekennzeichnet sind. Der Lehrenaufsatz 300 ist stufenlos verschieblich klemm- beziehungsweise vordefiniert absteckbar.

Der Lehrenaufsatz 300 ist durch die Stellschraube 350 feststellbar. Die Feststellvorrichtung, umfassend die Stellschraube 350, umfasst ferner eine Noniusskala zur Optimierung der Ablesbarkeit, wobei die Feststellvorrichtung zur analogen Fixierung des Lehrenaufsatzes 300 eingerichtet ist. Der Feststellmechanismus ist ähnlich dem eines Messschiebers, der mit der Stellschraube 350 realisiert ist, die je nach individueller Operationssituation beziehungsweise Operationsanatomie des Patienten stufenlose Zwischengrößen realisierbar macht.

Eine Absteckvorrichtung des Lehrenaufsatzes 300 durch den Abstecker 360 ermöglicht mehrere mögliche Absteckhöhen zur Anpassung des Lehrenaufsatzes 300. Beispielsweise ermöglichen spezielle Abstände wie 18, 20, 22, 24 mm, bezogen auf bereits in vorhergehenden Operationsschritten beispielsweise speziell festgelegte Maße wie ebenso 18, 20, 22, 24 mm, mehrere mögliche Absteckhöhen des Lehrenaufsatzes 300. Die Absteckung erfolgt durch den Abstecker 360, wobei eine falsche Einstellung durch eine spezielle Absteckergeometrie ausgeschlossen ist. Die jeweiligen Abstecköffnungen 325 können rechteckig in Form von Langlöchern ausgestaltet sein. Die Absteckergeometrie umfasst um jeweils 360° geteilt durch die Anzahl der Langlöcher gedrehte Langlöcher in dem Lehrenaufsatz 300 und den Führungselementen 120. Vorzugsweise sind Zahlenwerte zur Kenntlichmachung der auszuwählenden Position auf der Außenfläche des Lehrenaufsatzes 300 angebracht. Diese Absteckergeometrie ist nach der bekannten Methode "Poka Yoke" mit in diesem Fall geometrischen Einrichtungen gestaltet, die unbeabsichtigte Fehlstellungen vermeiden.

Die Bohrbuchse 308 in dem Lehrenaufsatz 300 ist integral in den Lehrenaufsatz 300 durch ausreichende Grundhärte des Materials integriert. Alternativ kann die Bohrbuchse als Bohrbuchseneinsatz ausgestaltet sein und dabei geklemmt, eingepresst oder andersartig in dem Lehrenaufsatz 300 befestigt sein.

Die Grundfläche 111 des Grundkörpers 110 ist zugunsten des Operationsablaufes optimiert. Hierbei ist die Geometrie der Grundfläche 111 des Grundkörpers 110 zugunsten der Vermeidung intraoperativer Kollisionen mit dem Kreuzband und/oder weiterem Gewebe verändert. Die Geometrie der Grundfläche 111 des Grundkörpers 110 ist beispielsweise konkav ausgeprägt und weist eine allgemeine Kantennachentschärfung (Radius, Fase oder Ähnliches) der Kontur zur Schnitt- und Abschabungsvermeidung von Gewebe und Knochen auf.

In einem Beispiel kann die Größe der Grundfläche 111 des Grundkörpers 110 aufgeteilt sein zugunsten eines Operationsablaufes und individueller Patientenanatomie, das heißt individueller Knochengröße. Beispielsweise kann der Grundkörper 110 in Prothesengröße 1 bis 6 und 8 bis 12, eventuell mit Größenüberdeckung, aufgeteilt sein. Die Grundfläche 111 liegt vorzugsweise planparallel und formschlüssig auf der Tibiastirnfläche 5 auf.

Die erfindungsgemäße Operationsvorrichtung 1 sowie sämtliche Komponenten und Elemente können aus unterschiedlichen Werkstoffen und gegebenenfalls mit unterschiedlichen Oberflächeneigenschaften beziehungsweise -beschichtungen ausgeführt sein. Sämtliche einzelne Elemente können in Metallausführung, in Kunststoffausführung oder in Hybridausführung, gegebenenfalls auch in Kombination und in Verbundbauweisen ausführbar sein. Auch müssen nicht alle Einzelteile aus dem gleichen Material gebildet sein, und Kombinationen unterschiedlicher Materialien zwischen unterschiedlichen Elementen sind ebenso möglich.

Die erfindungsgemäße Operationsvorrichtung 1 ist ausschließlich zur Optimierung des während der Knieendoprothesenoperation auftretenden Beugespaltes konzipiert. Dadurch, dass die gleichmäßige Spannung der Ligamente in einer gebeugten Stellung von Tibia zu Femur ermöglicht wird, ist eine momentenbalancierte Ausrichtung des Femurs erreichbar. Wird im Gegensatz dazu lediglich beim gebeugten Knie eine Parallelität von sich gegenüberliegenden Spannflächen zur Ausrichtung herangezogen, führt diese quasi parallel erzwungene Zwangslage des Femurs nicht zu einer gleichmäßigen Spannung der Ligamente sondern zu einer fehlerhaften Spannung.

Die erfindungsgemäße Operationsvorrichtung hat demnach gegenüber diesen parallel gespannten Ligamentausrichtungen den Vorteil, dass der Femur momentenbalanciert ausgerichtet werden kann, ohne dass der gesamte Operationsprozess durch Verwendung der integrierten Vorrichtung zusammen realisierbar ist.

Anstelle dessen, dass bisher für die Einzelschritte einzelne Vorrichtungsaufsätze benutzt werden müssen, liegt in dem Erkennen des Zusammenhangs zwischen festem Bohrungsabstand zur Unterkante der Vorrichtung bei gleichzeitigem Ablehren der Größe der Prothese mittels Abstecken der Größe durch Abstecker 360 oder Klemmschraube 350 und Integration in einer einzigen Vorrichtung die Verbesserung gegenüber dem bekannten Stand der Technik. Gegenüber den bekannten Vorrichtungen ermöglicht die erfindungsgemäße Vorrichtung 1 mit der einzigen kombinierten Ablehr- und Bohrkomponente 30 bei der Erzeugung des Beugespaltes die Schritte der Größenbestimmung der Prothese und die Bohrlöcher für den dazugehörigen Sägeblock zusammen in einer Einheit zu verwirklichen.

## Patentansprüche

1. Operationsvorrichtung (1) für eine Operation eines menschlichen Knies,
wobei die Operationsvorrichtung (1) eine Führungskomponente (10) und eine Spannkomponente (20) aufweist,
wobei die Führungskomponente (10) einen Grundkörper (110) mit einer Grundfläche (111) zur Anordnung auf einer Tibiastirnfläche (5) und sich von der Grundfläche (111) erstreckende Führungselemente (120) aufweist,
wobei die Spannkomponente (20) dazu eingerichtet ist, die Führungskomponente (10) derart zu spannen, dass die Ligamente des Knies in einem gebeugten Zustand des Knies gleichmäßig gespannt sind, wobei die Operationsvorrichtung eine Ablehr- und Bohrkomponente (30) zum Ablehren und Bohren einer Femurstirnfläche (4) aufweist, wobei die Ablehr- und Bohrkomponente (30) auf die Führungselemente (120) aufschiebbar und in verschiedenen Positionen bezüglich der Grundfläche (111) fixierbar ist, **dadurch gekennzeichnet, dass** die Ablehr- und Bohrkomponente (30) einen Lehrenaufsatz (300) aufweist, wobei der Lehrenaufsatz (300) wenigstens eine Nut (302) für einen oberen Femurschnitt aufweist, wobei der Lehrenaufsatz (300) mehrere Durchgangsöffnungen (325) aufweist und ein Führungselement (120) mehrere jeweils zugehörige Aussparungen (125) aufweist, wobei einander zugehörige Durchgangsöffnungen (325) und Aussparungen (125) die gleiche Orientierung aufweisen und wobei voneinander verschiedene Durchgangsöffnungen (325) voneinander verschiedene Orientierungen aufweisen.

2. Operationsvorrichtung (1) nach Anspruch 1, wobei die Spannkomponente (20) einen Spannzylinder (210) und einen Spannhebel (220) umfasst,
wobei der Spannzylinder (210) von den Führungselementen (120) entlang einer Längsachse des Spannzylinders (210) geführt ist und
wobei der Spannhebel (220) dazu eingerichtet ist, den Spannzylinder (210) bezüglich des Grundkörpers (110) zu spannen.

3. Operationsvorrichtung (1) nach Anspruch 1, wobei die Durchgangsöffnungen (325') auf einer Vorderseite des Lehrenaufsatzes (300) angeordnet sind, wobei die Vorderseite des Lehrenaufsatzes (300) dazu eingerichtet ist, während der Verwendung der Operationsvorrichtung (1) von der Femurstirnfläche (4) abgewandt zu sein.

4. Operationsvorrichtung (1) nach einem der Ansprüche 1 oder 3, wobei der Lehrenaufsatz (300), mittels eines Stellelementes, insbesondere mittels einer Klemmschraube (350), in einer variablen Höhenlage zur Grundfläche (111) feststellbar ist.

5. Operationsvorrichtung (1) nach einem der Ansprüche 2 bis 4, wobei der Spannzylinder (210) eine Aussparung (214) in Längsrichtung, insbesondere eine irgendwie geartete Vertiefung oder eine Durchgangsbohrung in Längsrichtung, aufweist.

6. Operationsvorrichtung (1) nach einem der Ansprüche 2 bis 5, wobei der Spannhebel (220) einen ersten Arm (221) und einen zweiten Arm (222) mit einem dazwischen liegenden Drehpunkt (223) aufweist, die derart eingerichtet sind, dass der zweite Arm (222) durch eine Vorspannung des ersten Arms (221) gegen den Spannzylinder (210) gespannt wird.

7. Operationsvorrichtung (1) nach einem derAnsprüche 2 bis 6, wobei der Grundkörper (110) auf der Grundfläche (111) eine Aussparung (116) zur Aufnahme des Spannhebels (220) aufweist, wobei der Grundkörper (110) insbesondere auf einer Seitenfläche (112) davon eine Öffnung (116) zur Betätigung des Spannhebels (220) aufweist.

8. Operationsvorrichtung (1) nach einem der vorstehenden Ansprüche, wobei die Führungskomponente (10) zwei voneinander beabstandete Führungselemente (120) aufweist, die sich von dem Grundkörper (110) in der gleichen Richtung erstrecken, wobei die beiden Führungselemente (120) je eine innere Seitenfläche (122), die dem jeweils anderen der Führungselemente (120) zugewandt ist, und eine der inneren Seitenfläche (122) gegenüberliegende Außenfläche (124) aufweisen, wobei insbesondere jede der inneren Seitenflächen (122) eine konkave zylindrische Form aufweist, derart, dass die beiden inneren Seitenflächen (122) mit dem dazwischenliegenden Raum eine zylindrische Führung (130) definieren.

9. Operationsvorrichtung (1) nach Anspruch 8, wobei in Erstreckungsrichtung der Führungselemente (120) eine der Außenflächen (122) eine Vertiefung (126) und die andere der Außenflächen (124) einen Vorsprung (128) zur Führung einer Ablehr- und Bohrkomponente (30) aufweist.

10. Operationsvorrichtung (1) nach Anspruch 8 oder 9, wobei die Außenflächen (124) der Führungselemente (120) eine Schwalbenschwanzführung zur Führung der Ablehr- und Bohrkomponente (30) bilden.

11. Operationsvorrichtung (1) nach einem der vorstehenden Ansprüche, wobei der Lehrenaufsatz (300) zwei Bohrbuchsen (308) zum Ausführen der Femurprothesenbohrungen aufweist, wobei die Bohrbuchsen (308) integral in dem Lehrenaufsatz (300) integriert oder als Bohrbuchseneinsatz ausgeführt sind, insbesondere geklemmt oder eingepresst sind.

12. Operationsvorrichtung (1) nach einem der vorstehenden Ansprüche, wobei der Lehrenaufsatz (300) eine Millimeterskala (307) auf seiner Vorderseite aufweist, die durch eine Kennzeichnung (129) auf der Führungskomponente (10) referenziert wird.

## Claims

1. A surgery device (1) for operating on the human knee,
the surgery device (1) comprising a guide component (10) and a tensioning component (20),
the guide component (10) comprising a base body (110) having a base surface (111) for disposing on a tibial end face (5) and comprising guide elements (120) extending from the base surface (111),
the tensioning component (20) being configured for tensioning the guide component (10) such that the ligaments of the knee are uniformly tensioned in a flexed state of the knee,
wherein the surgery device comprises a truing and drilling component (30) for truing and drilling a femoral end face (4), wherein the truing and drilling component (30) can slide on the guide elements (120) and can be fixed at various positions relative to the base surface (111),
**characterized in that** the truing and drilling component (30) comprises a gage fixture (300), the gage fixture (300) comprises at least one groove (302) for an upper femoral cut, the gage fixture (300) comprises a plurality of pass-through openings (325) and a guide element (120) comprises a plurality of associated recesses (125), wherein pass-through openings (325) and recesses (125) associated with each other have the same orientation and wherein pass-through openings (325) different from each other have orientations different from each other.

2. The surgery device (1) according to claim 1, wherein the tensioning component (20) comprises a tensioning cylinder (210) and a tensioning lever (220),
wherein the tensioning cylinder (210) is guided by the guide elements (120) along a longitudinal axis of the tensioning cylinder (210), and
wherein the tensioning lever (220) is set up for tensioning the tensioning cylinder (210) relative to the base body (110).

3. The surgery device (1) according to claim 1, wherein the pass-through openings (325') are disposed on a front side of the gage fixture (300), wherein the front side of the gage fixture (300) is set up for being faced away from the femoral end face (4) during the use of the surgery device (1).

4. The surgery device (1) according to any one of the claims 1 or 3, wherein the gage fixture (300) can be set at a variable height position relative to the base surface (111) by means of a setting element, particularly by means of a clamping screw (350).

5. The surgery device (1) according to any one of the claims 2 through 4, wherein the tensioning cylinder (210) comprises a cutout (214) in the longitudinal direction, particularly a depression or through hole of any kind in the longitudinal direction.

6. The surgery device (1) according to any one of the claims 2 through 5, wherein the tensioning lever (220) comprises a first arm (221) and a second arm (222) having a pivot point (223) present between said arms and set up such that the second arm (222) is tensioned against the tensioning cylinder (210) by a pretensioning of the first arm (221).

7. The surgery device (1) according to any one of the claims 2 through 6, wherein the base body (110) comprises a recess (116) on the base surface (111) for receiving the tensioning lever (220), wherein the base body (110) comprises, in particular on a side surface (112) thereof, an opening (116) for actuating the clamping lever (220).

8. The surgery device (1) according to any one of the preceding claims, wherein the guide component (10) comprises two guide elements (120) spaced apart from each other which are extending in the same direction from the base body (110), wherein the two guide elements (120) each comprise one inner lateral surface (122) facing toward the other one of the guide elements (120) and one outer surface (124) opposite the inner lateral surface (122), wherein in particular each of the inner lateral surfaces (122) comprises a concave cylindrical shape, such that the two inner lateral surfaces (122) define a cylindrical guide (130) by means of the intermediate space.

9. The surgery device (1) according to claim 8, wherein one of the outer surfaces (122) comprises a recess (126) and the other one of the outer surfaces (124) comprises a protrusion (128) in the direction of extent of the guide elements (120) for guiding a truing and drilling component (30).

10. The surgery device (1) according to any one of the claims 8 or 9, wherein the outer surfaces (124) of the guide elements (120) form a dovetail guide for guiding the truing and drilling component (30).

11. The surgery device (1) according to one of the preceding claims, wherein the gage fixture (300) comprises two drill bushings (308) for drilling the femoral prosthesis holes, wherein the drill bushings (308) are integrated in the gage fixture (300) or are implemented as drill bushing inserts, particularly clamped or pressed in.

12. The surgery device (1) according to any one of the preceding claims, wherein the gage fixture (300) comprises a millimeter scale (307) on the front side thereof referenced by a marking (129) on the guide component (10).

## Revendications

1. Dispositif chirurgical (1) pour une opération sur un genou humain,
dans lequel le dispositif chirurgical (1) comprend un composant de guidage (10) et un élément de serrage (20),
ledit composant de guidage (10) comprenant un corps de base (110) ayant une surface de base (111) pour le placement sur une surface d'extrémité tibiale (5) et des éléments de guidage (120) s'étendant à partir de ladite surface de base (111),
ledit élément de serrage (20) étant configuré pour tendre le composant de guidage (10) de telle sorte que les ligaments du genou soient uniformément tendus dans un état fléchi du genou,
ledit dispositif chirurgical comprend un composant de calibrage et de perçage (30) pour calibrer et percer une surface d'extrémité du fémur (4), le composant de calibrage et de perçage (30) pouvant coulisser sur les éléments de guidage (120) et pouvant être fixé dans différentes positions par rapport à la surface de base (111),
**caractérisé en ce que** le composant de calibrage et de perçage (30) comprend un embout de calibrage (300), l'embout de calibrage (300) ayant au moins une rainure (302) pour une coupe fémorale supérieure, l'embout de calibrage (300) ayant une pluralité d'ouvertures traversantes (325) et un élément de guidage (120) ayant une pluralité d'évidements (125), dans lequel les ouvertures traversantes (325) et les évidements (125) associés les uns aux autres ont la même orientation et dans lequel les ouvertures traversantes (325) mutuellement différents ont des orientations mutuellement différentes.

2. Dispositif chirurgical (1) selon la revendication 1, dans lequel l'élément de serrage (20) comprend un cylindre de serrage (210) et un levier de serrage (220),
dans lequel le cylindre de serrage (210) est guidé par les éléments de guidage (120) le long d'un axe longitudinal du cylindre de serrage (210), et
dans lequel le levier de serrage (220) est adapté pour serrer le cylindre de serrage (210) par rapport au corps de base (110).

3. Dispositif chirurgical (1) selon la revendication 1, dans lequel lesdites ouvertures traversantes (325') sont disposées sur un côté avant de ledite embout de calibrage (300), ledit côté avant de ledite embout de calibrage (300) étant adapté pour être tourné à l'opposé de ladite surface d'extrémité du fémur (4) pendant l'utilisation dudit dispositif chirurgical (1).

4. Dispositif chirurgical (1) selon l'une des revendications 1 ou 3, dans lequel l'embout de calibrage (300) peut être fixée dans une position en hauteur variable par rapport à la surface de base (111) au moyen d'un élément de réglage, en particulier au moyen d'une vis de serrage (350).

5. Dispositif chirurgical (1) selon l'une des revendications 2 à 4, dans lequel le cylindre de serrage (210) comporte un évidement (214) dans la direction longitudinale, en particulier un évidement quelconque ou un trou traversant dans la direction longitudinale.

6. Dispositif chirurgical (1) selon l'une des revendications 2 à 5, dans lequel le levier de serrage (220) comprend un premier bras (221) et un second bras (222) avec un point de pivotement (223) entre eux, disposés de telle sorte que le second bras (222) est serré contre le cylindre de serrage (210) par une tension préalable du premier bras (221).

7. Dispositif chirurgical (1) selon l'une des revendications 2 à 6, le corps de base (110) présentant sur la surface de base (111) un évidement (116) pour recevoir le levier de serrage (220), le corps de base (110) présentant en particulier sur une surface latérale (112) de celui-ci une ouverture (116) pour actionner le levier de serrage (220).

8. Dispositif chirurgical (1) selon l'une des revendications précédentes, dans lequel ledit composant de guidage (10) comprend deux éléments de guidage (120) espacés l'un de l'autre, s'étendant à partir dudit corps de base (110) dans la même direction, chacun desdits deux éléments de guidage (120) ayant une surface latérale intérieure (122) tournée vers l'autre desdits éléments de guidage (120), et une surface extérieure (124) opposée à la surface latérale intérieure (122), dans laquelle en particulier chacune des surfaces latérales intérieures (122) présente une forme cylindrique concave de telle sorte que les deux surfaces latérales intérieures (122) définissent un guide cylindrique (130) avec l'espace entre elles.

9. Dispositif chirurgical (1) selon la revendication 8, dans lequel, dans la direction d'extension des éléments de guidage (120), l'une des surfaces extérieures (122) présente un évidement (126) et l'autre des surfaces extérieures (124) présente une saillie (128) pour guider un composant de calibrage et de perçage (30).

10. Dispositif chirurgical (1) selon la revendication 8 ou 9, dans lequel les surfaces extérieures (124) des éléments de guidage (120) forment un guidage en queue d'aronde pour le guidage du composant de calibrage et de perçage (30).

11. Dispositif chirurgical (1) selon l'une des revendications précédentes, dans lequel l'embout de calibrage (300) présente deux douilles de perçage (308) pour exécuter le perçage des trous de la prothèse fémorale, les douilles de perçage (308) étant intégrées dans l'embout de calibrage (300) ou étant conçues comme des inserts de douilles de perçage, en particulier étant serrées ou enfoncées.

12. Dispositif chirurgical (1) selon l'une des revendications précédentes, dans lequel l'embout de calibrage (300) présente sur sa face avant une échelle millimétrique (307) référencée par un marquage (129) sur le composant de guidage (10).
